# EUROPEAN PATENT SPECIFICATION

(11) **EP 3 398 430 B1**
(45) Date of publication and mention of the grant of the patent: **18.09.2024**
(21) Application number: 18170224.2
(22) Date of filing: 01.05.2018
(51) Int. Cl.: A01H 5/10, C12N 9/02, C07D 489/02

(54) **MODIFIED POPPY PLANTS HAVING ELEVATED LEVELS OF THEBAINE**
MODIFIZIERTE MOHNPFLANZE MIT ERHÖHTEM GEHALT AN THEBAIN
PLANTE DE PAVOT MODIFIÉE A TENEUR ELEVEE EN THEBAINE

(30) Priority: 02.05.2017 GB 201706920; 03.11.2017 GB 201718241
(43) Date of publication of application: 07.11.2018
(73) Proprietor: Sun Pharmaceutical Industries Australia PTY Limited, Notting Hill, VIC 3168 (AU)
(72) Inventor: WINZER, Thilo, York, Yorkshire YO10 5DD (GB); GRAHAM, Ian, York, Yorkshire YO10 5DD (GB)
(74) Representative: Symbiosis IP Limited

(56) References cited:
- WO-A1-2009/109012
- WO-A1-2017/122011
- WO-A1-98/02033
- CA-A1- 2 941 315
- CHAMPA P. WIJEKOON ET AL: "Systematic knockdown of morphine pathway enzymes in opium poppy using virus-induced gene silencing", THE PLANT JOURNAL, vol. 69, no. 6, 28 March 2012 (2012-03-28), pages 1052 - 1063, XP055067993, ISSN: 0960-7412, DOI: 10.1111/j.1365-313X.2011.04855.x
- MILLGATE: "MORPHINE-PATHWAY BLOCK IN TOP1 POPPIES", NATURE, MACMILLAN JOURNALS LTD, LONDON, GB, vol. 431, 1 January 1996 (1996-01-01), pages 413 - 414, XP008075877, ISSN: 0028-0836, DOI: 10.1038/431413A
- JILLIAN M HAGEL ET AL: "Dioxygenases catalyze the O-demethylation steps of morphine biosynthesis in opium poppy", NATURE CHEMICAL BIOLOGY, vol. 6, no. 4, 14 March 2010 (2010-03-14), pages 273 - 275, XP055090844, ISSN: 1552-4450, DOI: 10.1038/nchembio.317

## Description

### Field of the invention

The disclosure relates to a *Papaver somniferum* plant that comprises deletion of all or part of three codeine 3-O-demethylase genes (CODM) and which have no expression of codeine 3-O-demethylase wherein said modified *Papaver somniferum* plant is further modified by deletion of all or part of five thebaine 6-O-demethylase genes (T6ODM) which do not express thebaine 6-O-demethylase and said plant is characterised by an increased thebaine content when compared to an unmodified *Papaversomniferum* plant.

### Background to the Invention

Opiate alkaloids like codeine or morphine are potent analgesics and are frequently used to treat moderate to strong pain. However, although having excellent analgesic properties, codeine and morphine cause often severe side effects and include vomiting, constipation or itchiness. Semisynthetic opioids such as oxycodone, oxymorphone, nalbuphine, naloxone, naltrexone, buprenorphine or etorphine have often a lower side effect profile thus offering a valuable pain-management-alternative to morphine or codeine.

The biosynthetic pathway of morphinan alkaloids is well established and includes a series of enzymatic and non-enzymatic steps starting from dopamine and 4-hydroxyphenylacetaldehyde leading to the synthesis of R-reticuline which is subsequently transformed to thebaine. Thebaine is then converted to either oripavine by the codeine 3-O-demethylase (CODM) or transformed in several steps to codeine involving the activity of the thebaine-6-demethylase (T6ODM). Codeine is subsequently converted to morphine by demethylation.

As pointed out above thebaine is the starting material for the synthesis of most semisynthetic opioids. Whilst opiate alkaloids can be extracted from latex, harvested from the green seed pods of opium poppy or from the poppy straw which is the dried mature plant, the demand for the opiate alkaloids in general exceeds the available natural supply necessitating costly chemical synthesis. Especially thebaine the chemical precursor of codeine, morphine and semisynthetic opioids is readily converted in the plant and thus the abundance is in general low.

Opium poppy mutants with increased alkaloid content have been obtained through random mutagenesis resulting in the disruption of the biosynthetic pathways leading to the different morphinans. US2013205452 discloses mutagenized Papaver somniferum poppy plants with thebaine as predominant alkaloid and substantially no oripavine, morphine or codeine. The plants were randomly mutagenized and subsequently selected for the high thebaine phenotype. CA2941315 discloses high thebaine opium poppy plants with substantially no oripavine, morphine or codeine content which are genetically modified to reduce the activity of the T6ODM and CODM. Plants were transformed with an expression construct comprising a nucleic acid molecule targeting both the CODM and T6ODM by RNAi. Transgenic plants with a concentration of up to 8.28 wt percent thebaine per 100 mg dry capsule were achieved. However, although gene silencing is a very effective method to knock out genes temporarily, it can be unstable over multiple generations and therefore unsuitable to generate consistent phenotypes.

Mutagenesis of the germplasm through chemical mutagens or radiation creates a diverse range of modified alleles or deletion mutants leading to an altered and stable phenotype which is often unobtainable when using traditional plant breeding techniques or through gene silencing. The applicants identified in their co-pending application various CODM genes linked on a gene cluster carrying polymorphisms linked to a high codeine phenotype in *Papaver somniferum* plants. Targeted mutagenesis or deletion of one or more copies of the CODM results in plants with high codeine in the poppy straw and reduced levels of morphine. The codeine concentration was further increased when the whole gene cluster carrying the CODM copies was deleted.

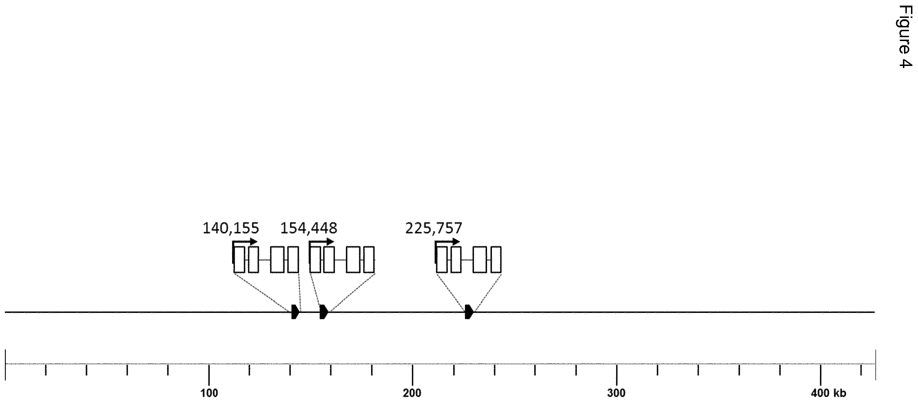

In our PCT application published as WO2017/122011 we disclose the presence of multiple copies of CODM genes and a region of the *Papaver somniferum* genome that includes a CODM gene cluster comprising at least 3 CODM genes. Plants modified in this genomic region by partial or entire deletion of a 426.6KB region, as illustrated in Figure 4, results in plants that do not express detectable CODM activity suggesting that the genes encoding these enzymes are either deleted or non-functional. The plants so modified have elevated levels of codeine. When plants of this phenotype are crossed with a plant with a high thebaine and oripavine phenotype the resultant plants obtained from the cross have a phenotype that is substantially no oriparvine, morphine or codeine but high levels of thebaine.

### Statement of the Invention

According to an aspect of the invention there is provided a *Papaver somniferum* plant wherein the plant is modified and comprises:
a genomic deletion to all or part of three genes encoding codeine 3-O-demethylases,
a genomic deletion to all or part of five genes encoding thebaine 6-O-demethylases,
wherein the expression or activity of said three codeine 3-O-demethylases is undetectable and further wherein the expression or activity of said five thebaine 6-O-demethylases is undetectable wherein the modified plant has a thebaine content that is at least 90% of the total extracted alkaloid content of latex or dried straw when compared to a wild type *Papaver somniferum* plant comprising functional genes encoding codeine 3-O-demethylases and thebaine 6-O-demethylases.

Genomic modifications include addition, deletion or substitution of one or more nucleotides or one or more nucleic acids in the intron or exon sequences of the gene, in the untranslated regions such as 5' and 3' untranslated regions and in regulatory elements such as promoters, transcription factors or response elements controlling gene transcription. The invention also encompasses mutations or deletions in regulatory genes such as transcription factors that regulate transcription of codeine 3-O-demethylases and/or thebaine 6-O-demethylase(s).

In a preferred embodiment of the invention said genomic modification comprises deletion of at least 50% of the nucleotide sequence set forth in SEQ ID NO: 1 wherein said modification deletes all or part of said at least two, three or more codeine 3-O-demethylase genes.

In a preferred embodiment of the invention said genomic modification comprises deletion of at least 55%, 60%, 65%, 70%, 80%, 90%, 95%, 96%, 97%, 98% or 99% of the nucleotide sequence set forth in SEQ ID NO: 1 wherein said modification deletes all or part of said five codeine 3-O-demethylase genes.

In a preferred embodiment of the invention said genomic modification comprises deletion of the nucleotide sequence set forth in SEQ ID NO: 1 wherein said *Papaver* plant is deleted for each functional copy of said codeine 3-O-demethylase genes.

In a preferred embodiment of the invention said genomic modification comprises deletion of at least 50% of the nucleotide sequence set forth in SEQ ID NO: 25 wherein said modification deletes all or part of thebaine 6-O-demethylase genes.

In a preferred embodiment of the invention said genomic modification comprises deletion of at least 55%, 60%, 65%, 70%, 80%, 90%, 95%, 96%, 97%, 98% or 99% of the nucleotide sequence set forth in SEQ ID NO: 25 wherein said modification deletes all or part of said at least two, three, four or five, of the thebaine 6-O-demethylase genes.

In a preferred embodiment of the invention said genomic modification comprises deletion of the nucleotide sequence set forth in SEQ ID NO: 25 wherein said *Papaver* plant is deleted for five thebaine 6-O-demethylase genes.

A thebaine 6-O-demetylase or codeine 3-O-demetylase gene means genes which encode polypeptides with thebaine 6-O-demethylase or codeine 3-O-demethylase activity. The term "gene" thus excludes partial genes or pseudogenes which comprise for example a premature stop codon or mutation resulting in either the expression of an inactive protein or no protein expression.

In a preferred embodiment of the invention said codeine 3-O-demethylases and/or thebaine 6-O-demethylase expression or activity is undetectable.

In a preferred embodiment of the invention said Papaver plant is modified wherein said modification is to a genomic region comprising SEQ ID NO: 1 and SEQ ID NO: 25 wherein said modification deletes three codeine 3-O-demethylase and five thebaine 6-O-demethylase genes wherein expression or activity of codeine 3-O-demethylase and thebaine 6-0-demethylase is undetectable.

In a preferred embodiment of the invention the *Papaver somniferum* plant comprises a thebaine content that is at least 90% wt of the total extracted alkaloid content of latex or dried straw.

Preferably the Papaver *somniferum* plant comprises a thebaine content that is at least 91%, 92%, 93%, 94%, 95%, 96%, 97%, 98% or 99 % wt of the total extracted alkaloid content of latex or dried straw.

In a preferred embodiment of the invention the *Papaver somniferum* plant comprises a thebaine content that is at least 90% wt of the total extracted alkaloid content of latex or dried straw in noscapine minus plants.

Preferably the Papaver *somniferum* plant comprises a thebaine content that is at least 91%, 92%, 93%, 94%, 95%, 96%, 97%, 98% or 99 % wt of the total extracted alkaloid content of latex or dried straw in noscapine minus plants.

"Total alkaloid content" is defined relative to the major alkaloids: morphine, codeine, noscapine, oripavine and thebaine.

In noscapine minus plants, e.g. plants lacking the genes responsible for noscapine synthesis the "Total alkaloid content" is defined relative to the sum of the major alkaloids: morphine, codeine, oripavine and thebaine.

Dried straw refers to the dried stalks, stem and leaves of poppies minus the seeds.

In an alternative embodiment of the invention the *Papaver somniferum* plant comprises a thebaine content of at least 1.0 wt % of the dried straw of said modified plant.

Preferably the thebaine content of said *Papaver somniferum* plant is between 1.0 wt % and 10.0 wt % of the dried straw; or between 5.0 wt % and 6.0 wt % of the dried straw.

Preferably the thebaine content of the *Papaver somniferum* plant is about 5.7 wt % of the dried straw.

According to a further aspect of the invention there is provided a seed obtained or obtainable from a *Papaver somniferum* plant according to the invention.

According to a further aspect of the invention there is provided a seed pod obtained or obtainable from a *Papaver somniferum* plant according to the invention.

According to a further aspect of the invention there is provided dried straw obtained or obtainable from the *Papaver somniferum* plant according to the invention.

According to an aspect of the invention there is provided a process for the extraction of thebaine from *aPapaver somniferum* plant comprising the steps:
i) harvesting a plant or plant material prepared from a *Papaver somniferum* plant according to the invention;
ii) forming a reaction mixture of particulate plant material;
iii) solvent extraction of the reaction mixture to provide an alkaloid enriched fraction; and
iv) concentrating said alkaloid enriched fraction to provide a thebaine enriched fraction.

In a preferred method of the invention said plant material comprises poppy straw and/or poppy latex.

Throughout the description and claims of this specification, the words "comprise" and "contain" and variations of the words, for example "comprising" and "comprises", means "including but not limited to", and is not intended to (and does not) exclude other moieties, additives, components, integers or steps. "Consisting essentially" means having the essential integers but including integers which do not materially affect the function of the essential integers.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

An embodiment of the invention will now be described by example only and with reference to the following figures and tables:

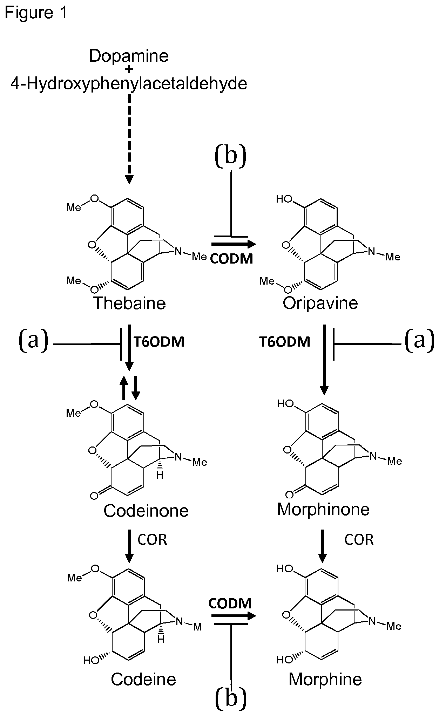

Figure 1: Biosynthesis of morphinan alkaloids in opium poppy (*Papaver somniferum* L.)The first morphinan alkaloid, thebaine, is synthesised through a series of enzymatic and non-enzymatic conversions starting from dopamine and 4-hydroxyphenylacetaldehyde. Thebaine is further converted by enzymatic and non-enzymatic reactions to morphine. In thebaine/oripavine material T6ODM activity is blocked (a), whereas in codeine material CODM activity has been abolished by deletion of the CODM gene cluster (b). In thebaine-only material the block of the T6ODM activity has been combined with the deletion of the CODM gene cluster (a + b);
   Dashed arrows indicate multiple enzymatic and non-enzymatic conversions. Black arrows indicate enzymatic or non-enzymatic conversions. The T-shaped symbol indicated a block of enzymatic conversions. T6ODM, Thebaine 6-O-Demethylase; Codeine 3-O-Demethylase, CODM; Codeinone reductase, COR;

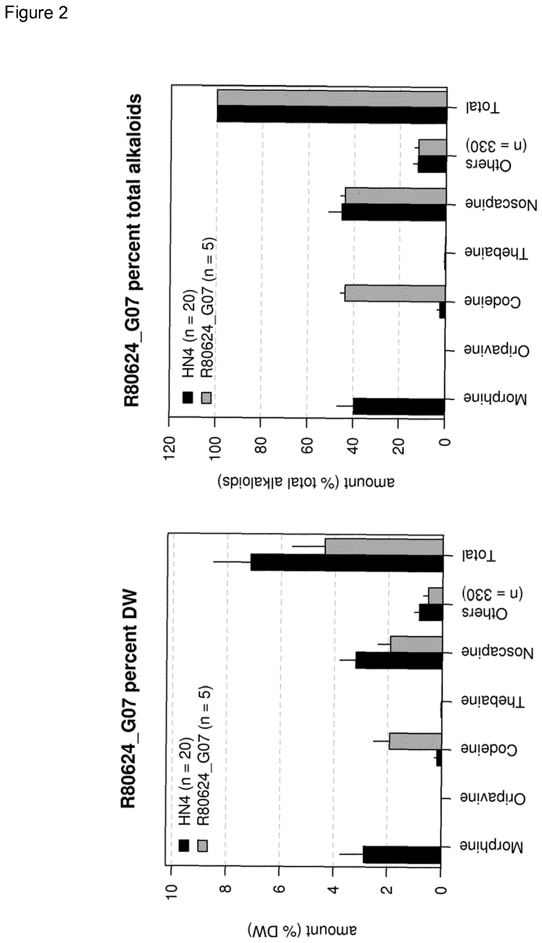

Figure 2: Absolute and relative alkaloid content in M3 capsules of the fast neutron mutagenised high codeine mutant R80624_G07 compared to control plants of the non-mutagenised wild type cultivar HN4. DW, dry weight;

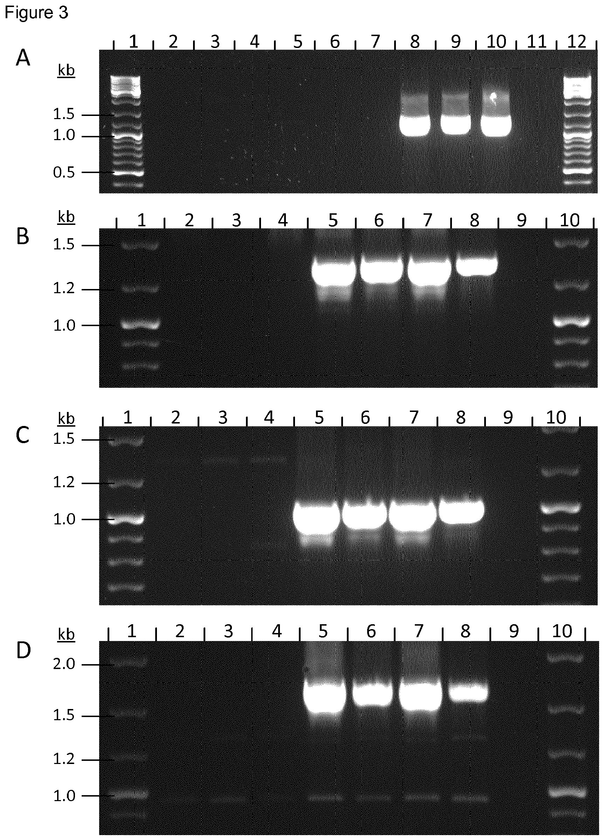

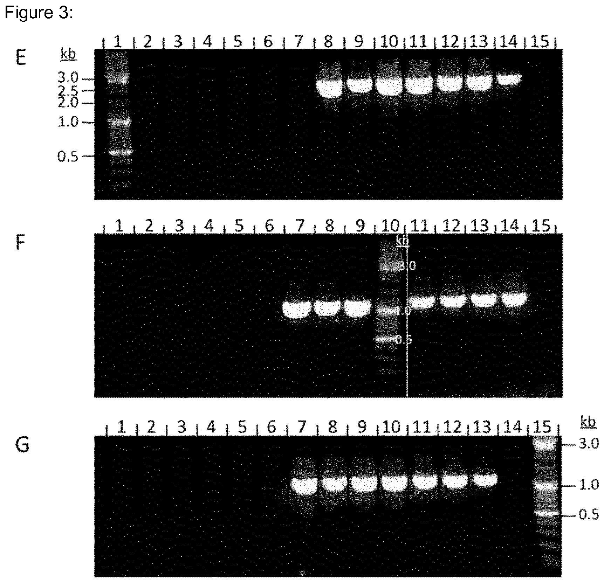

Figure 3: Amplification of CODM fragments from M4 individuals of the fast neutron mutagenised codeine/noscapine mutant line and from non-fast neutron mutagenised control plants; A, primer pair 178/179, expected fragment size: 1296 bp; lane 1 and 12, size marker (GeneRuler DNA Ladder Mix, Thermo Fisher Scientific); lanes 2-7, M4 mutant plants Sd-701292, Sd-701293, Sd-701294, Sd-701302, Sd-701303, Sd-701304; lanes 8-10, control plants Sd-763815, Sd-763818, Sd-863819; lane 11, no template control. B, pimer pair 178/181, expected fragment size: 1347 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. C, pimer pair 180/179, expected fragment size: 1016 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. D, primer pair 180/168, expected fragment size: 1659 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. E, primer pair 167/168, expected fragment size: 2330 bp; lane 1, size marker (as above); lanes 2-8, loss of M4 mutant plants Sd-701292, Sd-701293, Sd-701294, Sd-701302, Sd-701303, Sd-701304; lanes 8-14, control plants Sd-763815, Sd-763818, Sd-863819, Sd-764849, Sd-764851, Sd-764857, Sd-106305; lane 15, no template control. F, primer pair 175/176, expected fragment size: 1122 bp; lane 10, size marker (as above); lanes 1-6, M4 mutant plants Sd-701292, Sd-701293, Sd-701294, Sd-701302, Sd-701303, Sd-701304; lanes 7- 9, control plants Sd-763815, Sd-763818, Sd-863819; lanes 11- 14 control plants Sd-764849, Sd-764851, Sd-764857, Sd-106305; lane 15, no template control. G, primer pair 175/177, expected fragment size: 1122 bp; lane 15, size marker (as above); lanes 1-7, M4 mutant plants Sd-701292, Sd-701293, Sd-701294, Sd-701302, Sd-701303, Sd-701304; lanes 7-13, control plants Sd-763815, Sd-763818, Sd-863819, Sd-764849, Sd-764851, Sd-764857, Sd-106305; lane 14, no template control;
Figure 4: Illustrates the genomic arrangement of CODM genes in opium poppy. The structure and position of the three CODM genes are shown above the central black line, which represents 426.6 kb of genomic sequence. Exons are represented by solid grey boxes and introns by fine black lines. Arrows indicate the 5' to 3' orientation of each gene. The number above the gene structure indicates the start position of each gene (first base of transcriptional start codon) within the genomic sequence comprising the CODM genes (SEQ ID NO: 1);

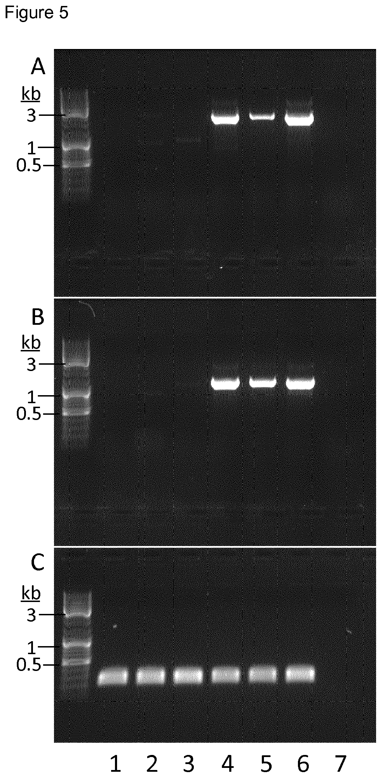

Figure 5: Amplification of CODM fragments from BC1F2 individuals segregating the CODM gene cluster deletion mutation in a thebaine/oripavine opium poppy background. A, primer pair 167/168, expected fragment size: 2230 bp; B, pimer pair 178/179, expected fragment size: 1296 bp; C, Actin control primers (Actin_F/-_R), expected fragment size: 202 bp; lane 1, size marker (GeneRuler DNA Ladder Mix, Thermo Fisher Scientific); lanes 2-4, BC1F2 plants homozygous for the CODM gene cluster deletion allele; lanes 5-7, BC1F2 plants not homozygous for the CODM gene cluster deletion allele; lane 8, no template control;

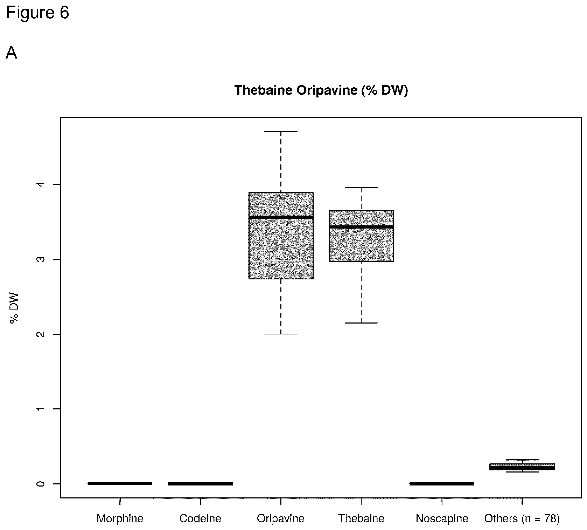

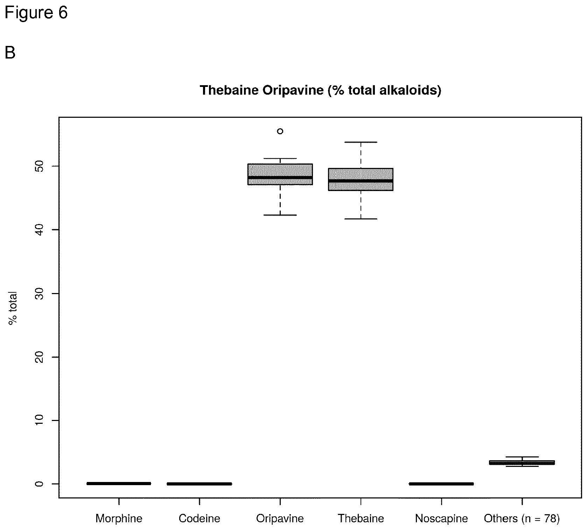

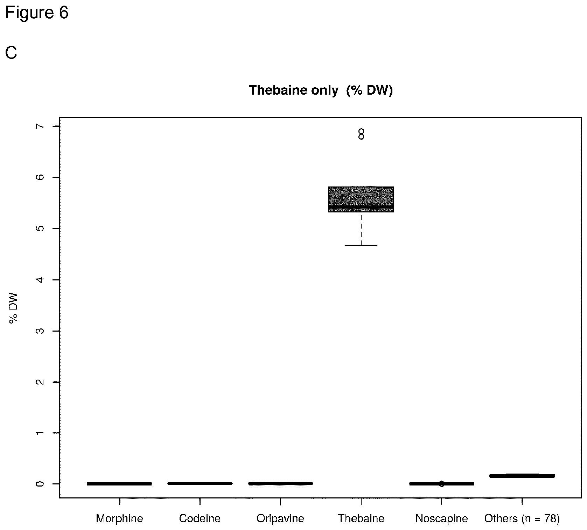

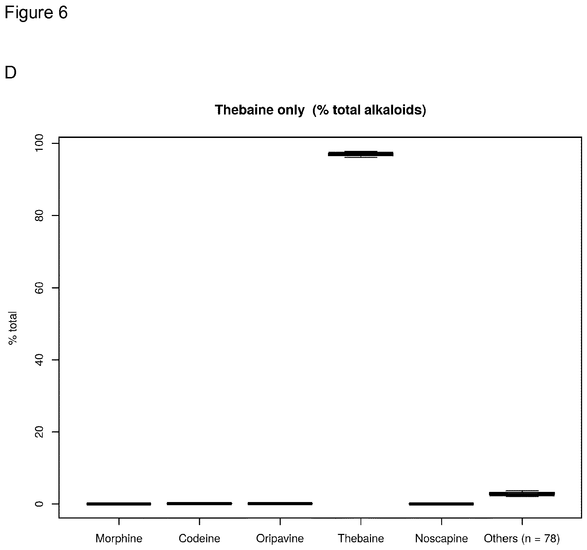

Figure 6: Absolute and relative amounts of major morphinan alkaloids in dried capsules from thebaine/oripavine and thebaine only material. Absolute amounts are expressed as percentage of dry weight (DW). Relative amounts are expressed as percentage total alkaloids including non-morphinan alkaloids. Alkaloid data are shown for individuals (n=6) of the thebaine/oripavine variety serving as recurrent parent for introgression of the CODM gene cluster deletion allele and the BC₁F₂ individuals (n=3) shown in Figure 5 to be homozygous for the CODM gene cluster deletion allele. A, Major alkaloid content (% DW) of the thebaine/oripavine variety serving as recurrent parent for introgression of the CODM gene cluster deletion allele; B, relative alkaloid content (% total alkaloids) of the thebaine/oripavine variety serving as recurrent parent for introgression of the CODM gene cluster deletion allele; C, Major alkaloid content (% DW) of BC₁F₂ plants homozygous for the CODM gene cluster deletion allele ('thebaine only' material); D, relative alkaloid content (% total alkaloids) ) of BC₁F₂ plants homozygous for the CODM gene cluster deletion allele (`thebaine only' material);

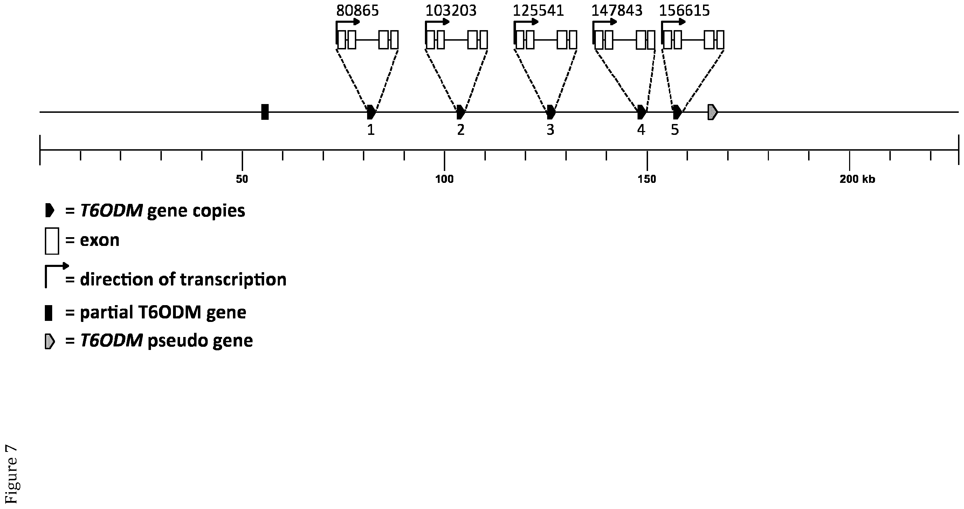

Figure 7: Genomic arrangement of *T6ODM* gene copies in opium poppy. The position of the *T6ODM* genes are shown as pointed boxes on a central black line, which represents 227.26 kb of genomic sequence. The structures of the five *T6ODM* genes are shown as enlargement above the central black. Exons are represented by solid grey boxes and introns by fine black lines. Arrows indicate the 5' to 3' orientation of each gene. The number above the gene structure indicates the start position of each gene (first base of transcriptional start codon). In addition to the five functional genes the T6ODM gene cluster is flanked by a partial T6ODM gene represented by a black box and a pseudogene represented by a grey pointed box. The pseudogene contains one premature STOP codon; and

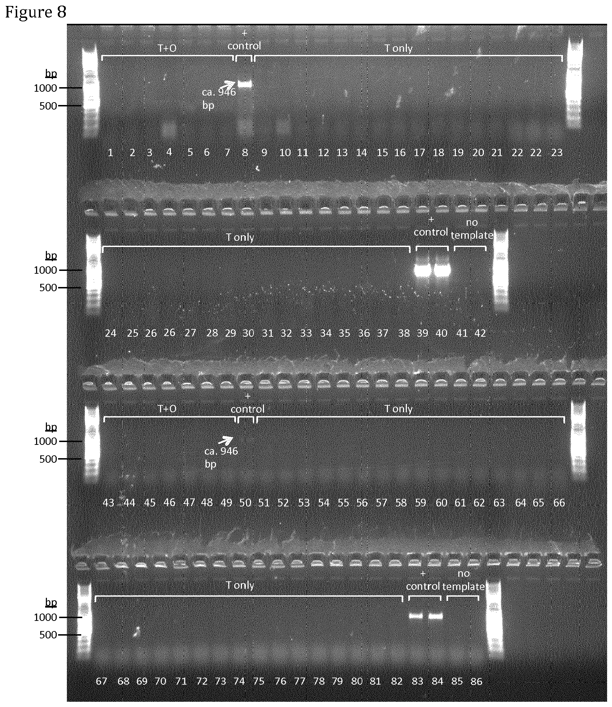

Figure 8: Amplification of *T6ODM* fragments from thebaine/oripavine (T/O) plants and plants accumulating thebaine as the sole dominant morphinan alkaloid (T). Lanes 1-42: primer pair A (Table 7 and 8); this primer pair amplifies fragments from *T6ODM* genes 1-3; expected fragment size 946 pb (*T6ODM* genes 1 and 2) and 945 bp (*T6ODM* gene 3). Lanes 43-86: primer pair B (Table 7 and 8); this primer pair amplifies fragments from *T6ODM* genes 4 and 5; expected fragment size 923 bp (*T6ODM* gene 4) and 945 bp (*T6ODM* gene 5). Positive control: Sun Pharmaceuticals noscapine/morphine cultivar HN2. Size standard = 5µl of GeneRuler^{™} (Thermo Scientific).

### Materials and Methods

### Fast Neutron Deletion Mutagenesis and glasshouse-based forward screening

Fast neutron mutagenesis (FNM) was carried out by the HAS Centre for Energy Research, Radiation Protection Department, H-1121 Budapest, Konkoly Thege út 29-33, X. epulet, Hungary. About 40 g of seed of the Sun Pharmaceutical Industries (Australia) cultivar High Noscapine 4 (HN4) were exposed to 20 Gy (beam time 50 min). The M1 generation was self-pollinated. One M2 plant per M2 seed family was grown. Latex was collected from 9 week old M2 seedlings and analysed. M2 plants displaying an altered alkaloid composition in the latex compared to HN4 controls were grown to maturity to confirm the phenotype in dry M2 capsule material. M3 seed was collected from M2 plants with confirmed phenotypes. Five M3 plants were then grown per M2 mutant plant to confirm the heritability of the phenotype in mature dry M3 capsule material. The M1 to M3 generations were all grown in the glasshouse.

### Leaf latex and dry capsule analysis of glasshouse grown material

Latex was collected from 9 week old plants from cut petioles, with a single drop dispersed into 500 µL of 10% acetic acid. This was diluted 10× in 1% acetic acid to give an alkaloid solution in 2% acetic acid for further analysis. Capsules were harvested from the same plants used for latex analysis and single capsules were ground to a fine powder in a ball mill (Model MM04, Retsch, Haan, Germany). Samples of ground poppy straw were then weighed accurately to 10 ±0.1 mg and extracted in 0.5 ml of a 10% acetic acid solution with gentle shaking for 1h at room temperature. Samples were then clarified by centrifugation and a 50 µl subsample diluted 10× in 1% acetic acid to give an alkaloid solution in 2% acetic acid for further analysis.

All solutions were analysed using a Waters Acquity UPLC system (Waters Ltd., Elstree, UK) fitted with a Waters Acquity BEH C18 column, 2.1 mm × 100 mm with 1.7 micron packing. The mobile phase used a gradient profile with eluent A consisting of 10 mM ammonium bicarbonate of pH 10.2 and eluent methanol. The mobile phase gradient conditions used are as listed in the table below with a linear gradient. The flow rate was 0.5 ml per minute and the column maintained at 60°C. The injection volume was 2 µl and eluted peaks were ionised in positive APCI mode and detected within 5 ppm mass accuracy using a Thermo LTQ-Orbitrap. The runs were controlled by Thermo Xcalibur software (Thermo Fisher Scientific Inc., Hemel Hempstead,UK).

| **TIME (minutes)** | **% Eluent A** | **% Eluent B** | **Flow (mL/min)** |
|---|---|---|---|
| 0.0 | 98 | 2 | 0.50 |
| 0.2 | 98 | 2 | 0.50 |
| 0.5 | 60 | 40 | 0.50 |
| 4.0 | 20 | 80 | 0.50 |
| 4.5 | 20 | 80 | 0.50 |

All data analysis was carried out using the R programming language and custom scripts. Standards for morphine, oripavine, codeine, thebaine and noscapine were used to identify and quantify these alkaloids using exact mass, retention time and peak areas for the pseudomolecular ion. Other putative alkaloid peaks were quantified by their pseudomolecular ion areas relative to the thebaine standard. For putative alkaloids, the Bioconductor rcdk package (Guha, (2007) J.Stat. Software 6(18)) was used to generate pseudomolecular formulae from exact masses within elemental constraints C = 1 100, H = 1 200, O = 0 200, N = 0 3 and mass accuracy < 5 ppm. The hit with the lowest ppm error within these constraints was used to assign a putative formula.

### Bacterial Artificial Chromomsome (BAC) library construction and screening

High Noscapine cultivar cvs 1 was used to generate a Bacterial Artificial Chromomsome (BAC) library as described in Winzer et al. ((2012), Science 336:1704-8). A 703 bp fragment located in the CODM promoter region (Raymond M, "Isolation and characterization of latex-specific promoters from Papaver somniferum", Master thesis, Virginia Polytechnic Institute and State University, Blacksburg,VA) was amplified with primers AAAATCCGCCCTCCATGC (forward) (SEQ ID NO 26) and CCGACTTTGGCCCACTTGT (SEQ ID NO 27) (reverse) using a PCR digoxigenin (DIG) synthesis kit (Roche Applied Science, Indianapolis, IN) according to the manufacturer's instruction to obtain the DIG-labelled screening probe. Screening of the BAC library was performed as described Winzer et al. ((2012), Science 336:1704-8) and resulted in the identification of 10 positive BAC clones, namely BAC33_D07, BAC86_F04, BAC89_C05, BAC109_H06, BAC129_K11, BAC152_G13, BAC158_A11, BAC185_L02, BAC195_N12 and BAC230_D02.

For screening the BAC library for T6ODM containing BACs a screening probe was generated as described above using primers CCGAGATTAAGGGTATGTCAGAGG (forward) (SEQ ID NO 28) and CACAAGATCCCCATATGTATATCCAC (reverse) (SEQ ID NO 29). Amplification with these primers would generate screening probe fragments between 502 to 526 bp (depending on the T6ODM gene amplified) corresponding to the 3' end of the genes. Screening of the BAC library was performed as described Winzer et al. ((2012), Science 336:1704-8) and resulted in the identification of 5 positive BAC clones, namely BAC 30_E04, BAC70_J09, BAC70_P15, BAC81_K11, BAC127_B22.

### BAC Sequencing and Sequence Assembly

Each BAC was sequenced on two sequencing platforms: Paired-end sequencing was performed on the Illumina HiSeq platform (Illumina,Inc, San Diego, CA).

In addition, each BAC clone was sequenced using a MinION sequencer (Oxford Nanopore Technologies Ltd, Oxford, UK). Purified BACs were minimally fragmented using 20 second treatments with NEBNext^{®} dsDNA Fragmentase^{®} (New England Biolabs Inc., Ipswich, MA), and sequencing libraries prepared using Oxford Nanopore Technologies sequencing kit SQK007 with native barcoding expansion pack EXP-NDB002. Briefly, single stranded nicks in DNA were repaired using NEBNext^{®} FFPE DNA repair mix prior to end repair and dA tailing using the NEBNext^{®} Ultra^{™} II End Repair/dA-Tailing Module. Unique barcode sequences were ligated onto fragments for each BAC, before pooling 3-4 BACs per library. Sequencing adapters (including a hairpin adapter to allow for 2D sequencing) were ligated onto the ends of fragments, along with an adapter-binding tether protein. Fragments where tether protein was bound were purified using MyOne^{™} C1 steptavidin beads (Invitrogen/Thermo Fisher Scientific, UK). Libraries were then run on MinION R9 flowcells using a 48 hour sequencing protocol, and base calling and demultiplexing was perfomed using Metrichor's EPI2ME platform. Reads that passed 2D quality checks were split according to barcode for further analysis.

### Sequence Assembly

Raw MiniON reads of each BAC clone were assembled with CANU v1.3 software (Koren et al. (2016) bioRxiv, doi.org/10.1101/071282) and insert boundaries were identified with the positions of vector sequences and cross-reference of overlapping BAC clones. These initial assemblies were further corrected with the NANOPOLISH software. The high quality Illumina paired-end short reads were then mapped with BWA software (Li and Durbin (2009) Bioinformatics, 25: 1754-60) to the resulting MinION assemblies, which were used as the scaffolding reference. Consensus reference were generated with the mapped alignment and indels were corrected according to the variation analyses of the alignment. The corrected consensus was then used as a new reference in a reiterative process until no further improvement could be achieved. This assembly method has shown to give a 99.2 % base identity to a previously assembled and published BAC sequence (BAC164_F07, gene bank accession: JQ659012). The overlapping BACs were then combined to give a continuous 426 KB CODM genome fragment.

### Preparation of genomic DNA

For genomic DNA extraction 30-50 mgs of leaf material was collected from 4-6 week old glasshouse-grown seedlings. Genomic DNA was extracted using the BioSprint 96 Plant kit on the BioSprint 96 Workstation (Qiagen, Crawley, UK) according to the manufacturer's protocol. Extracted DNA was spectrometrically quantified on the NanoDrop^{®} (Thermo Scientific, UK) and normalized to 5 or 10 ng/ul.

### Amplification of CODM fragments from genomic DNA

CODM fragment PCR amplifications were carried out on genomic DNA from M4 individuals derived from the high codeine/noscapine fast neutron mutagenised mutant material R80624_G07 (M4 mutant siblings: Sd-701292, Sd-701293, Sd-701294, Sd-701302, Sd-701303, Sd-701304; control plants Sd-763815, Sd-763818, Sd-863819, Sd-764849, Sd-764851, Sd-764857, Sd-106305) as well as on non-fast neutron mutagenised material derived from Sun Pharmaceutical Industries (Australia) morphine cultivar HM2.

PCR reactions containing 5x GoTaq Flexi buffer, 0.2mM dNTPs, 1.0 µM forward and reverse primer (Tables 5 and 6), 1U GoTaq Flexi DNA polymerase (Promega, M8305), 1.5 mM MgCl₂ and 10 ng of template DNA in a total volume of 10µl were run on a Tetrad thermocycler (Bio-Rad) under the following conditions: 94°C for 2 minutes, followed by 10 cycles of 94°C for 15 seconds, 65°C decreasing to 60°C by 0.5°C per cycle for 30 seconds, 72°C for 1-1.5 minute (depending on fragment length), followed by 25 cycles of 94°C for 15 seconds, 60°C for 30 seconds, 72°C for 1-1.5 minute (depending on fragment length), followed by 72°C for 10 minutes, then a hold at 7°C. PCR products were analysed on 1-1.5% agarose gels. Primers and primer combinations used for amplification are shown in Tables 5 and 6.

### Amplification of T6ODM fragments from genomic DNA

T6ODM fragment PCR amplifications were carried out on genomic DNA extracted from thebaine/oripavine material and material accumulating thebaine as the sole dominant morphinan alkaloids. DNA extracted from Sun Pharmaceutical Industries (Australia) morphine/noscapine cultivar HN2 served as positive control. PCR reactions containing 1x GoTaq Flexi buffer, 0.125 mM dNTPs, 1.0 µM forward and reverse primer, 0.5U GoTaq Flexi DNA polymerase (Promega, M8305), 1.5 mM MgCl₂ and 10 ng of template DNA in a total volume of 10µl were run on a Tetrad thermocycler (Bio-Rad) under the following conditions: 94°C for 2 minutes, followed by 35 cycles of 94°C for 30 seconds, 65°C for 30 seconds, 72°C for 1 minute followed by 72°C for 4 minutes, then a hold at 7°C. PCR products were analysed on 1% agarose gels. Primer sequences and primer combinations used for amplification are shown in Tables 7 and 8.

**Table 5: Primers used for fragment amplification**

| **Primer_id** | **sequence (5'->3')** |
|---|---|
| 167 (SEQ ID 30) | GTGGTTGCTCAGACCCTTCGT |
| 168(SEQ ID 31) | AGCACCACCACCAAGCCTTC |
| 175(SEQ ID 32) | AACCATGGAGTCGACGCTTT |
| 176(SEQ ID 33) | TCTCTGGTGTGACCAAGCTC |
| 177(SEQ ID 34) | TCTCTGGTGTGACCAAGCTA |
| 178(SEQ ID 35) | CTCACGCTTGCAGAAATTCC |
| 179(SEQ ID 36) | CTCGACGCTACGGTAAATCC |
| 180(SEQ ID 37) | GTTAACCATGGAGTCGACGC |
| 181(SEQ ID 38) | AAATGTCGCGATTGAGAGCC |
| Actin_F (SEQ ID 39) | GGGTACTCATTCACCACCACT |
| Actin_R (SEQ ID 40) | GGTTGGAAAAGCACCTCTGG |

**Table 6: Primer combination for amplification of genomic CODM fragments shown in Figure 3 and 5**

| Fragment | Forward primer ID | Reverse primer ID | Expected fragment size | Figure |
|---|---|---|---|---|
| 1 | 178 | 179 | 1296 | Figure 3A and 5B |
| 2 | 178 | 181 | 1347 | Figure 3B |
| 3 | 180 | 179 | 1016 | Figure 3C |
| 4 | 180 | 168 | 1659 | Figure 3D |
| 5 | 167 | 168 | 2330 | Figure 3E and 5A |
| 6 | 175 | 176 | 1122 | Figure 3F |
| 7 | 175 | 177 | 1122 | Figure 3G |

**Tabla 7: Primer IDs and sequences for amplification of T6ODM fragments**

| Primer ID | Orientation | sequence (5'->3') |
|---|---|---|
| 308(SEQ ID 41) | Forward | ACACCTAAGTCTCCCTATTTCTGC |
| 309(SEQ ID 42) | Forward | ACACCTAAGTCTCCCTATTTCTAT |
| 313(SEQ ID 43) | Reverse | ATAGTTGAATCTGACATGCAATCAC |

**Table 8: Primer pairs used for amplification of T6ODM gene fragments shown in Figure 8.**

| Primer pair | Forward Primer ID | Reverse Primer ID | T6ODM genes amplified | Expected fragment size [bp] | Shown in Figure 8 |
|---|---|---|---|---|---|
| A | 308 | 313 | 1,2,3 | 946 (*T6ODM* genes 1 and 2); | Lanes 1 - 42 |
| | | | | 945 (*T6ODM* gene 3) | |
| B | 309 | 313 | 4, 5 | 923 (*T6ODM* gene 4); | Lanes 43 - 86 |
| | | | | 945 (*T6ODM* gene 5) | |

**Table 9: Sequence identification**

| SEQ ID No | |
|---|---|
| 1 | Genomic region comprising the CODM genes (SEQ ID NO 2-8) |
| 2-8 | CODM genes |
| 9 | T6ODM variant |
| 10-14 | Genomic sequences of T6ODM variants |
| 15-19 | cDNA sequences of T6ODM variants |
| 20-24 | Polypeptide sequences of the T6ODM variants displayed in SEQ ID NO 15-19 |
| 25 | Genomic region comprising the T6ODM genes (SEQ ID NO 10-14) |
| 26-43 | Primer |

### Plant Material

Plants for crossing and phenotyping were grown in Rootrainers^{™} (Haxnicks, Mere, UK) under glass in 16 hour days at the University of York horticulture facilities. The growth substrate consisted of 4 parts John Innes No. 2, 1 part Perlite and 2 parts Vermiculite.

### Introgression of the CODM gene cluster deletion allele into thebaine/oripavine material lacking T6ODM catalysed conversions

Fast neutron mutagenised high codeine/no morphine M4 material harbouring the CODM gene cluster deletion allele was introgressed into a recurrent parent variety stably displaying the thebaine/oripavine phenotype such as disclosed in WO2009109012. The thebaine/oripavine phenotype displayed a Mendelian inheritance consistent with a single recessive locus being responsible (see below). The resulting F1 material was heterozygous for the recessive alleles blocking T6ODM activity and the CODM gene cluster deletion blocking CODM activity. F1 plants were backcrossed to the recurrent parent to generate BC₁ F₁ material.

50% of BC₁ F₁ plants displayed the thebaine/oripavine trait consistent with the block of T6ODM activity being caused by a single recessive locus. BC₁F₁ plants displaying the thebaine/oripavine trait were selected for self-pollination. In addition to being homozygous for the locus affecting the block of T6ODM activity 50% of these plants were expected to be heterozygous for the CODM gene cluster deletion allele. Thus 50% of the resulting BC₁F₂ populations would segregate the CODM gene cluster deletion.

BC₁F₂ plants homozygous for the CODM gene cluster deletion were selected by means of presence or absence PCR of the CODM genes from segregating BC₁F₂ populations (Figure 5). After selection, the alkaloid content and profile of dry capsules from BC₁F₂ plants homozygous for the CODM gene cluster deletion was analysed (Figure 6).

The fast neutron high codeine mutant material that served as the donor of the CODM gene cluster deletion allele did accumulate noscapine (Figure 2). The capacity for noscapine synthesis depends on the presence of the noscapine gene cluster (Winzer et al. (2012) Science 336, 1704-1708). The noscapine gene cluster did segregate independently of the CODM gene cluster deletion allele and could thus be removed in the course of backcrossing. The BC₁F₂ plants homozygous for the CODM gene cluster deletion allele shown in Figure 5 and 6 lacked the noscapine gene cluster and the ability to synthesise noscapine.

### CRISPR/cas9-mediated mutation or deletion of CODM and T6ODM gene copies

Genome editing and engineering technologies such as zinc finger nucleases (ZFNs) or transcription-activator-like effector nucleases (TALENs) or clustered regularly interspaced short palindromic repeats (CRSIPR) system allow genetic modifications to be made in a sequence-specific manner. Of these the CRSIPR system has emerged as the one of the most versatile and widely applicable systems. Modifications and changes of the canonical type II CRISPR/Cas9 system are ever expanding the range of sequences that can be targeted and the versatility of the system with applications now ranging from gene knock outs, control of gene expression (activation/repression), chromatin modifications among others.

### Deletion of CODM and/or T6ODM gene copies can be achieved by CRISPR/cas9-mediated mutation or deletion.

The Clustered Regularly Interspaced Short Palindromic Repeat (CRISPR)-Cas9 endonuclease systems allows RNA-guided genome modification. CRISPR systems comprise typically a guide RNA (or sgRNA) and a CRISPR-associated endonuclease (Cas9 endonuclease). The guide RNA comprises several elements consisting of a scaffold sequence required for Cas9 binding and a target specific sequence to be modified. In order to obtain target specific modification which can result in gene knock out, editing, interference or activation, the target specific sequence must be unique and adjacent to a Protospacer Adjacent Motif (PAM) Sequence.

### Selection of CODM and T6ODM target sequences for CRISPR/Cas9-mediated mutation or deletion

Nucleotide sequences suitable as guides were selected by scanning CODM and T6ODM gene copies for the Protospacer Adjacent Motif (PAM) nucleotide sequence `NGG'.

PAM sequences are specific to the species variants of Cas9. Several PAM sequences are known and selected form the group consisting of 3'NGG, 3'NGA, 3'NAG, 3'NGCG, 3'NGCG, 3'NGAG, 3'NGAN, 3'NGNG, 3'NNGRRT, 3'NNGRR(N), 5'TTTV, 5'TYCV, 5'TYCV, 5'TATV, 3'NNNNGATT, 3'NNAGAAW, 3'NAAAAC, 3'TTTN, wherein N is any nucleic acid, Y=C/T, V=A/C/G, W=A/T.

The twenty nucleotides upstream of identified PAMs were evaluated for their suitability for CRISPR/Cas9-mediated mutation or deletion based on the following criteria: Location in the first or second exon and absence of any polymorphisms between the gene copies in order to simultaneously target all copies of *T6ODM* and *CODM*, respectively.

Predicted target specificity of these pre-selected guide sequences was further evaluated by conducting BLASTN^{®} searches of transcriptomic and genomic sequence repositories such as those accessible through the National Center for Biotechnology Information (NCBI) and the 1KP transcriptome database (www.onekp.com). In addition, BLASTN^{®} searches of in-house transcriptomic and genomic data sets were conducted to identify putative off-target binding sites. Unique sequences (with either more than 4 mismatches to putative off-target sites or lacking PAM in off-targets) are shown in Table 10.

**Table 10: Target sequences selected for CRISPR/Cas9-mediated mutation or deletion**

| **Gene targeted** | **Target sequence** | **PAM** | **Location** |
|---|---|---|---|
| CODM | CCATCTCGATACACATGCAC (SEQ ID NO 44) | CGG | Exon 1 |
| | ATTATTCAACGGGCTTTCAC (SEQ ID NO 45) | CGG | Exon 1 |
| | TGGACAACCCTATATTGAAT (SEQ ID NO 46) | CGG | Exon 2 |
| T6ODM | TTCTATATCGATGACAGGAA (SEQ ID NO 47) | TGG | Exon 1 |
| | GGGAGTTTTGAAAATAAGTG (SEQ ID NO 48) | AGG | Exon 2 |

### CR!SPR-Cas9 constructs

### Single sgRNA CRISPR-Cas9 constructs

CRISPR-Cas9 constructs for the selected genomic targets can be obtained from Sigma's Ready-to-Use Cas9 and Guide RNA (gRNA) Expression Plasmid service (CRISPRPL, www.sigmaaldrich.com). This service provides plasmids with the user-defined genomic target sequence already incorporated into the gRNA cassette. The constructs are based on the type IIA CRISPR/Cas9 derived from *Streptococcus pyogenes.* There are a range of plasmid backbones to choose from depending on plant species, mode of plant transformation (*Agrobacterium*-mediated, ballistic, protoplast transformation), selective markers etc.

In the following there is described a method for CODM and *T6ODM* CRISPR/Cas9 mutation/deletion using binary plasmids for *Agrobacterium*-mediated transformation. In addition to the customized gRNA cassette, each plasmid contains a dicot U6 promoter for gRNA expression, a Cas9 coding sequence codon optimized for expression in dicots under the control of a 35S promoter and the bar gene (phosphinothricin acetyl transferase) to confer phosphinothricin (Basta^{®}) resistance to transformed plants. The total size of the plasmid is 13.5 kb (www.sigmaaldrich.com/technical-documents/articles/biology/genome-editing-in-plants-with-crispr-cas9.html#materials).

### Multiple sgRNA CRISPR-Cas9 constructs

### Assembly of multi sgRNA constructs for multiplexed CRISPR/Cas9 mutation induction

For the assembly of binary vector containing multiple sgRNA cassettes Golden Gate Molecular Cloning is used as essentially described by Engler et al. 2014, ACS Synth. Biol. 3: 839-843. Plasmids and Level 0 parts from the Golden Gate MoClo Plant Part kit (Addgene #1000000047), the Golden Gate MoClo Plant Toolkit (Addgene #1000000044) and the Golden Gate Plant CRISPR Kit (Sainsbury Laboratory, Norwich) are used to assemble plant selectable marker resistance gene expression cassettes (bar gene), Cas9 expression cassettes as well as multiple sgRNA units into one plasmid backbone for multiplex CRISPR/Cas9 genome editing.

sgRNA expression cassettes are assembled by amplifying the sgRNA scaffold from an sgRNA backbone containing plasmid (pICSL90010 or pICH86966::AtU6p::sgRNA_PDS, Addgene #46966) using primers with the guide sequences appended to the 5' tail of the primer. The forward primers have the following general sequence:
*tgtggtctca* ATTG NNNNN NNNNN NNNNN NNNNN gtttaagagctatgctggaaacag (SEQ ID NO 49).

The position of the guide sequence is indicated by N. The Bsal site is shown in italics, the overhang produced by Bsal digestion in capitals, 3' sequence shown in lower case is complementary to the sgRNA scaffold sequence. For guide sequences starting with a G, the 3'G of the Bsal overhang (G) was used as the 5' G of the guide sequence.

Forward Primers for generating the sgRNAs corresponding to the guide sequences shown in the table are as follows:
CODM
   tgt*ggtctca*ATTG CCATCTCGATACACATGCAC gtttaagagctatgctggaaacag (SEQ ID NO 50)
   tgt*ggtctca*ATTG ATTATTCAACGGGCTTTCAC gtttaagagctatgctggaaacag (SEQ ID NO 51)
   tgt*ggtctca*ATTG TGGACAACCCTATATTGAAT gtttaagagctatgctggaaacag (SEQ ID NO 52)
T6ODM
   tgt*ggtctca*ATTG TTCTATATCGATGACAGGAA gtttaagagctatgctggaaacag (SEQ ID NO 53)
   tgt*ggtctca*ATTG GGAGTTTTGAAAATAAGTG gtttaagagctatgctggaaacag (SEQ ID NO 54)

The reverse primer has the following sequence:
*tgtggtctct* AGCG aaaaaaagcaccgactcggtgccac(SEQ ID NO 55)

The Bsal site is shown in italics, the overhang produced by Bsal digestion in capitals, the 3' end in lower-case is complementary to the sgRNA scaffold sequence. The resulting amplicon is then assembled with the U6 RNA promoter from plasmid plCSL0002 (Addgene #68261) in an appropriate Level 1 acceptor. Level M and P constructs are assembled as described in Engler et al. 2014, ACS Synth. Biol. 3: 839-843.

The final constructs contain expression cassettes for the bar gene as well as a Cas9 expression cassette and either multiple sgRNA cassettes representing multiple guide sequences from *T6ODM* and *CODM* (Table x), respectively, to target *T6ODM* and *CODM* copies separately or for both gene families combined to target *T6ODM* and *CODM* copies simultaneously.

### Agrobacterium tumefaciens-Mediated Transformation

*Agrobacterium* tumefaciens-mediated transformations of elite opium poppy cultivars are performed according to the protocol established by Chitty et al. (2003, Functional Plant Biology, 30, 1045-1058). In summary, 3-6 mm hypocotyl segments of one-week old poppy seedlings are inoculated with *A. tumefaciens* strain (LBA4404) harbouring the respective constructs. After 3-4 days of co-culture the segments are transferred to selective callusing medium containing 10 mg/L phosphinothricin. Explants are transferred to fresh CM medium containing the selective agent every three weeks. Once embryogenic callus formation has occurred explants are transferred to B5 medium containing 10 mg/L phosphinothricin with transfer to fresh medium of the same composition every three weeks. Somatic embryos and, eventually, plantlets with roots form on the B5 medium which are transferred to F2 compost. The plants are maintained in a growth cabinet for a week before being transferred to the glasshouse. Propagator lids are initially kept over the plants and the vents slowly opened to allow the plants to harden off.

### Testing of transformants

T0 plants are genotyped for the presence of the T-DNA constructs harbouring CRISPR/Cas9 construct [PCR] and self-pollinated. The T1 progeny is assessed for the presence/absence of the T-DNA construct [PCR] as well a gene editing events in the CODM and T6ODM gene copies, respectively, by cloning and Sanger sequencing using sequence specific primers and PCR conditions as described above. Genotyping is accompanied by metabolite profiling of latex and dry capsule material as described above. T1 plants showing the metabolite profiles expected for total functional impairment of all copies of *T6ODM* and *CODM*, respectively, in the respective opium poppy genotypes are self-pollinated. Subsequent generations are analysed for the stable inheritance and zygosity of the Cas9-induced mutations/metabolite profile.

Genotyping and metabolite profiling of the T1 and subsequent generations may provide evidence for only partial functional impairment of the respective gene copies. This can be due to heterozygosity of Cas9-induced mutations and/or because not all copies of *CODM* and *T6ODM*, respectively, carry functionally disabling Cas9-induced mutations. In this case, additional rounds of self-pollination of T1 may be required to bring mutations to homozygosity, and/or (when using T1 plants harbouring the T-DNA construct) allow sgRNA-guided Cas9 activity to continue transgenerationally until all gene copies carry Cas9-induced disabling mutations.

Transgene-free plants harbouring stable and heritable Cas9-induced mutations can be obtained at various generations (T1, T2 etc) by segregating away the T-DNA construct (Lawrenson et al. 2015, Genome Biology, doi.org/10.1186/s13059-015-0826-7; Xu et al. 2015, Scientific Reports, doi.org/10.1186/s13059-015-0826-7).

In case of linkage between the inserted T-DNA construct and the targeted loci (*CODM* and *T6ODM* gene cluster, respectively) additional crosses to non-transformed poppy cultivars may be required to segregate the T-DNA construct away.

### Generating opium poppy plant material with thebaine as the sole major morphinan alkaloid using plants carrying Cas9-induced functionally disabling mutations in T6ODM and CODM copies.

Once material harbouring stable and heritable Cas9-induced mutations in all copies of *T6ODM* and *CODM*, respectively, has been generated, plants that accumulate thebaine as the sole major morphinan alkaloid ('thebaine-only' plants) are generated in a number of ways depending on the CRISPR/Cas9 constructs used, the opium poppy genotype used for transformation and opium poppy genotypes used for introgression:
a) Material resulting from transformations with T-DNA CRISPR/Cas9 constructs that simultaneously target all *T6ODM* and *CODM* copies: Homozygous plants with stable, heritable and functionally disabling Cas9-induced mutations in all *T6ODM* and *CODM* copies display 'thebaine-only' metabolite profile.
b) Material resulting from transformations with T-DNA CRISPR/Cas9 constructs that target *T6ODM* and *CODM* copies separately:
   Plants homozygous for Cas9-induced functionally disabling mutations in all *T6ODM* copies are crossed to plants homozygous for Cas9-induced functionally disabling mutations in all *CODM* copies. The resulting F1 generations is self-pollinated and plants homozygous for mutations in *T6ODM* and *CODM* copies selected from the progeny by genotyping and metabolite profiling. These plants display the 'thebaine-only' metabolite profile.

Alternatively, 'thebaine-only' material is obtained by crossing plants homozygous for functionally disabling Cas9-induced mutations in all *T6ODM* copies with material lacking functional *CODM* gene copies due to natural variation or mutagenesis such as the fast neutron mutagenesis material described above.

Alternatively, 'thebaine-only' material is obtained by crossing plants homozygous for functionally disabling Cas9-induced mutations in all *CODM* copies with material lacking functional *T6ODM* gene copies such as thebaine/oripavine material high codeine material described above.

c) Material resulting from transformations with T-DNA CRISPR/Cas9 constructs that target *T6ODM* copies using an opium poppy genotype for transformation which lacks functional *CODM* gene copies due to natural variation or mutagenesis such as the fast neutron mutagenesis material described above.

c) Material resulting from transformations with T-DNA CRISPR/Cas9 constructs that target *CODM* copies using an opium poppy genotype for transformation which lacks functional *T6ODM* copies due to mutagenesis or natural variation such as the thebaine/oripavine material described above.

### Example 1

### Multiple copies of CODM exist and are clustered in the genome of opium poppy

BAC sequencing and assembly revealed that Codeine demethylase (*CODM*) genes occur as a cluster of CODM genes within the opium poppy genome (Figure 4). Previously, the complementary DNA (cDNA) of just one *CODM* gene had been cloned (Hagel and Facchini, (2010) Nature Chemical Biology 6, 273-275).

### Example 2

The complete absence of CODM activity in a fast neutron mutagenised plant is caused by a deletion of all CODM genes leading to a complete loss of morphine and oripavine and gain of codeine.

Glasshouse-based forward screening of fast neutron mutagenised material of the high morphine and noscapine cultivar HN2 yielded a M2 mutant plant (R80624_G07) that completely lacked morphine and oripavine but had gained high levels of codeine indicating a complete loss of CODM activity. The plant had retained its capacity to synthesise noscapine. The M2 mutant plant was self-pollinated and the heritability of the codeine and noscapine phenotype was confirmed in M3 capsules (Figure 2). M3 plants contained on average 1.9 % dry weight each of codeine and noscapine which accounted in each case for nearly 44% of total alkaloids. The M3 generation, too, was self-pollinated. M4 progeny was grown and DNA isolated. Using a number of primers in various combinations failed to amplify any of the CODM genes in the M4 mutant plants whereas amplification was successful on DNA from non-fast neutron mutagenised morphine synthesising control plants (Figure 3). The complete lack of amplification of CODM genes suggests that the complete loss of CODM activity observed in capsule material derived from high codeine mutant R80624_G07 is a consequence of a deletion of all the CODM genes from its genome. This finding is consistent with the fact that the CODM genes occur as a cluster in the opium poppy genome. Fast neutron mutagenesis has been shown to be capable of inducing deletions ranging from several hundred to tens of thousands of base pairs (O'Rourke et al. (2013) Frontiers in Plant Science 4: Article 210). The lack of amplification of CODM fragments in the fast neutron mutagenised high codeine plants (Figure 3) indicates that the entire CODM gene cluster has been deleted. Thus morphinan alkaloid synthesis can proceed to codeine but conversion of codeine to morphine and from thebaine to oripavine is blocked in the mutant (Figures 1 and 2).

### Example 3

Introgression of the CODM gene cluster deletion allele into thebaine/oripavine material leads to opium poppy plants that accumulate thebaine as the sole dominant alkaloid while lacking other morphinan alkaloids.

The CODM gene cluster deletion mutation obtained by fast neutron mutagenesis was introgressed into material displaying a high thebaine and oripavine (lack of other morphinan alkaloids) phenotype.

An opium poppy mutant plant displaying the thebaine/oripavine phenotype was first described by Millgate et al. (2004), Nature 431, 413-414. The mutant plant was obtained by chemical mutagenesis and designated *top1* (for thebaine oripavine poppy 1). The *top1* mutant phenotype displayed Mendelian inheritance consistent with a single locus being responsible (Millgate et al. (2004), Nature 431, 413-414). Subsequently, additional opium poppy plants displaying a *top1*-like phenotype have been described and also shown to display Mendelian inheritance of a single, recessive locus (Hagel and Facchini, (2010) Nature Chemical Biology 6, 273-275).

Likewise, the thebaine/oripavine phenotype of the material used as recurrent parent for the introgression of the CODM gene cluster deletion allele displayed an inheritance consistent with a single recessive locus responsible for the phenotype. BC₁F₁ material that displayed the thebaine/oripavine phenotype (and thus lacking T6ODM catalysed conversions) was self-pollinated to generate BC₁F₂ populations segregating the CODM gene cluster allele.

BC₁F₂ plants homozygous for the gene cluster deletion allele were selected by means of CODM presence or absence PCR (Figure 5). These plants accumulate thebaine as the sole dominant morphinan alkaloid (Figure 6C and D) consistent with the plants being homozygous for the respective loci causing the metabolic blocks of T6ODM and CODM catalysed conversions (Figure 1). Thebaine levels in capsule material reached 5.69 ± 0.477 % on a dry weight basis. The relative amount of thebaine (percentage of total alkaloids measured including) reached 96.76 ± 0.61 %. Morphine was completely absent from the material. Remaining trace amounts of oripavine (0.0039 ± 0.0005 % on a dry weight basis, 0.067 ± 0.012 % total alkaloids) and codeine (0.0055 ± 0.0005 % on a dry weight basis, 0.095 ± 0.017 % total alkaloids) are likely to represent spontaneous demethylation of thebaine when thebaine accumulates to very high levels.

The discovery of multiple linked copies of *CODM* (*CODM* gene cluster) and the generation of a deletion allele of all *CODM* gene copies provides the molecular basis for achieving a phenotype with thebaine being the sole dominant morphinan alkaloid. The absence of all CODM genes and consequently of all CODM activity in a background devoid of T6ODM catalysed conversions is essential to achieving the thebaine only phenotype: Any remaining functional *CODM* gene would allow metabolic flux to proceed from thebaine to oripavine and codeine to morphine (Figure 1).

### Example 4

### Multiple copies of T6ODM exist and are clustered in the genome of opium poppy

BAC sequencing ted to the assembly of 227.26 kb of genomic sequence containing a cluster of multiple T6ODM demethylase (*T6ODM*) genes (Seq ID 25 and Figure 7). Previously, the complementary DNA (cDNA) of just one *T6ODM* gene had been cloned (Hagel and Facchini, (2010) Nature Chemical Biology 6, 273-275). There are at least five *T6ODM* gene copies contained within 70 kb of the genomic sequence. They are flanked upstream by a partial T6ODM gene copy and downstream by a non-functional *T6ODM* pseudogene (Figure 7).

### Example 5

### All T6ODM genes are deleted in thebaine/oripavine material as well as in material accumulating thebaine as the sole major morphinan alkaloid.

Using primers specific to the *T6ODM* gene 1 to 3 and to copies 4 and 5, respectively, failed to amplify fragments of any of the *T6ODM* genes from DNA obtained from plants displaying the thebaine/oripavine phenotype (Figure 8). Thus, all *T6ODM* genes are deleted in thebaine/oripavine material. The deletion of all *T6ODM* genes establishes the molecular basis for the thebaine/oripavine phenotype. Likewise, using the same primer combination failed to amplify any *T6ODM* genes from DNA obtained from BC₁F₂ plants resulting from the introgression of the *CODM* gene cluster deletion into thebaine/oripavine material (plants that accumulated thebaine as the sole major morphinan alkaloid as described in Example 3) failed to amplify any of the *T6ODM* genes. *T60DM* fragments of the expected size were amplified from DNA obtained from control plants (Sun Pharmaceuticals cultivar HN2) that did produce morphine. The deletion of all *T6ODM* genes in combination with the deletion of the *CODM* gene cluster (Examples 2 and 3) establishes the molecular basis of the thebaine-only phenotype (thebaine being the sole dominant morphinan alkaloid (Figure 1).

### Abstract

The disclosure relates to a plant of the genus *Papaver* somniferum that comprises deletion of all or part of three genes encoding codeine 3-O-demethylase (CODM) and which do not express detectable codeine 3-O-demethylase activity wherein said modified plant is further by deletion of all or part of five thebaine 6-O-demethylase genes (T6ODM) which do not express thebaine 6-O-demethylase activity, said plant is characterised by increased thebaine content when compared to an unmodified *Papaver* somniferum.

## Claims

1. A *Papaver somniferum* plant wherein the plant is modified and comprises:
a genomic deletion to all or part of three genes encoding codeine 3-O-demethylases,
a genomic deletion to all or part of five genes encoding thebaine 6-O-demethylases, wherein the expression or activity of said three codeine 3-O-demethylases is undetectable and further wherein the expression or activity of said five thebaine 6-O-demethylases is undetectable wherein the modified plant has a thebaine content that is at least 90% of the total extracted alkaloid content of latex or dried straw when compared to a wild type *Papaver somniferum* plant comprising functional genes encoding codeine 3-O-demethylases and thebaine 6-O-demethylases.

2. The *Papaver somniferum* plant according to claim 1 wherein said genomic deletion comprises deletion of all or part of the nucleotide sequence set forth in SEQ ID NO: 1 wherein said deletion comprise all or part of said three codeine 3-O-demethylase genes.

3. The *Papaver somniferum* plant according to claim 2 wherein said genomic deletion comprises deletion of at least 50% of the nucleotide sequence set forth in SEQ ID NO: 1 wherein said deletion is all or part of said three codeine 3-O-demethylase genes.

4. The *Papaver somniferum* plant according to any one of claims 1 to 3 te8- wherein said genomic deletion comprises deletion of at least 50% of the nucleotide sequence set forth in SEQ ID NO: 25 wherein said modification deletes all or part of said five or more thebaine 6-O-demethylase genes.

5. The *Papaver somniferum* plant according to claim 4 wherein said genomic modification comprises deletion of the nucleotide sequence set forth in SEQ ID NO: 25 wherein said *Papaver somniferum* plant is deleted for five thebaine 6-O-demethylase genes.

6. The *Papaver somniferum* plant according to claim 5 wherein the *Papaver* plant comprises a thebaine content that is at least, 91%, 92%, 93%, 94%, 95%, 96%, 97%, 98% or 99 % of the total extracted alkaloid content of latex or dried straw.

7. The *Papaver somniferum* plant according to any one of claims 1 to 6 wherein the Papaver plant comprises a thebaine content of at least 1.0 wt % of the dried straw of said modified plant.

8. The *Papaver somniferum* plant according to claim 7 wherein the thebaine content of said Papaver plant is between 1.0 wt % and 10.0 wt % of the dried straw.

9. The *Papaver somniferum* plant according to claim 8 wherein the thebaine content of said Papaver plant is between 5.0 wt % and 6.0 wt % of the dried straw.

10. The *Papaver somniferum* plant according to claim 9 wherein the thebaine content of said Papaver plant is about 5.7 wt % of the dried straw.

11. A seed or seed pod obtained or obtainable from a *Papaver somniferum* plant according to any one of claims 1 to 10.

12. Dried straw obtained or obtainable from the *Papaver somniferum* plant according to any one of claims 1 to 10.

13. A process for the extraction of thebaine from a *Papaver somniferum* plant comprising the steps:
i) harvesting a plant or plant material from a plant according to any one of claims 1 to 10;
ii) forming a reaction mixture of particulate plant material;
iii) solvent extraction of the reaction mixture to provide an alkaloid enriched fraction; and
iv) concentrating said alkaloid enriched fraction to provide a thebaine enriched fraction.

14. The process according to claim 13 wherein said plant material comprises poppy straw.

15. The process according to claim 13 wherein said plant material comprises poppy latex.

## Patentansprüche

1. *Papaver somniferum-Pflanze,* wobei die Pflanze modifiziert ist und umfasst:
eine genomische Deletion aller oder eines Teils von drei Genen, die Codein-3-O-Demethylasen kodieren,
eine genomische Deletion aller oder eines Teils von fünf Genen, die Codein-6-O-Demethylasen kodieren,
wobei die Expression oder Aktivität der fünf Thebain-6-O-Demethylasen nicht nachweisbar ist, wobei die modifizierte Pflanze einen Thebain-Gehalt aufweist, der wenigstens 90 % des gesamten extrahierten Alkaloidgehalts von Latex oder getrocknetem Stroh beträgt, verglichen mit einer *Papaver somniferum-Pflanze* des Wildtyps, die funktionelle Gene umfasst, die Codein-3-O-Demethylasen und Thebain-6-O-Demethylasen kodieren.

2. *Papaver somniferum*-Pflanze nach Anspruch 1, wobei die genomische Deletion die Deletion der gesamten oder eines Teils der in SEQ ID NO: 1 dargelegten Nukleotidsequenz umfasst, wobei die Deletion alle oder einen Teil der drei Codein-3-O-Demethylase-Gene umfasst.

3. *Papaver somniferum*-Pflanze nach Anspruch 2, wobei die genomische Deletion die Deletion von wenigstens 50 % der in SEQ ID NO: 1 dargelegten Nukleotidsequenz umfasst, wobei die Deletion alle oder einen Teil der drei Codein-3-O-Demethylase-Gene umfasst.

4. *Papaver somniferum*-Pflanze nach einem der Ansprüche 1 bis 3, wobei die genomische Deletion die Deletion von wenigstens 50 % der in SEQ ID NO: 25 dargelegten Nukleotidsequenz umfasst, wobei die Modifikation alle oder einen Teil der fünf oder mehr Thebain-6-O-Demethylase-Gene deletiert.

5. *Papaver somniferum*-Pflanze nach Anspruch 4, wobei die genomische Modifikation die Deletion der in SEQ ID NO: 25 dargelegten Nukleotidsequenz umfasst, wobei die *Papaver somniferum-*Pflanze für fünf Thebain 6-O-Demethylase-Gene deletiert ist.

6. *Papaver somniferum*-Pflanze nach Anspruch 5, wobei die *Papaver-*Pflanze einen Thebain-Gehalt aufweist, der wenigstens 91 %, 92 %, 93 %, 94 %, 95 %, 96 %, 97 %, 98 % oder 99 % des gesamten extrahierten Alkaloidgehalts von Latex oder getrocknetem Stroh beträgt.

7. *Papaver somniferum*-Pflanze nach einem der Ansprüche 1 bis 6, wobei die Papaver-Pflanze einen Thebain-Gehalt von wenigstens 1,0 Gew.-% des getrockneten Strohs der modifizierten Pflanze aufweist.

8. *Papaver somniferum*-Pflanze nach Anspruch 7, wobei der Thebain-Gehalt der Papaver-Pflanze zwischen 1,0 Gew.-% und 10,0 Gew.-% des getrockneten Strohs liegt.

9. *Papaver somniferum*-Pflanze nach Anspruch 8, wobei der Thebain-Gehalt der Papaver-Pflanze zwischen 5,0 Gew.-% und 6,0 Gew.-% des getrockneten Strohs beträgt.

10. *Papaver somniferum*-Pflanze nach Anspruch 9, wobei der Thebain-Gehalt der Papaver-Pflanze etwa 5,7 Gew.-% des getrockneten Strohs beträgt.

11. Samen oder Samenschoten, gewonnen oder gewinnbar aus einer *Papaver somniferum*-Pflanze nach einem der Ansprüche 1 bis 10.

12. Getrocknetes Stroh, gewonnen oder gewinnbar aus der *Papaver somniferum*-Pflanze nach einem der Ansprüche 1 bis 10.

13. Verfahren zur Extraktion von Thebain aus einer *Papaver somniferum*-Pflanze, umfassend die Schritte:
i) Ernten einer Pflanze oder von Pflanzenmaterial von einer Pflanze nach einem der Ansprüche 1 bis 10;
ii) Bilden eines Reaktionsgemisches aus partikulärem Pflanzenmaterial;
iii) Lösungsmittelextraktion des Reaktionsgemisches zum Bereitstellen einer alkaloidangereicherten Fraktion; und
iv) Konzentrieren der alkaloidangereicherten Fraktion zum Bereitstellen einer thebainangereicherten Fraktion.

14. Verfahren nach Anspruch 13, wobei das Pflanzenmaterial Mohnstroh umfasst.

15. Verfahren nach Anspruch 13, wobei das Pflanzenmaterial Mohnlatex umfasst.

## Revendications

1. Plante *Papaver somniferum* dans laquelle la plante est modifiée et comprend :
une délétion génomique de tout ou partie de trois gènes codant pour des codéine 3-O déméthylases,
une délétion génomique de tout ou partie de cinq gènes codant pour des codéine 6-O déméthylases,
dans lequel l'expression ou l'activité desdites cinq thébaïnes 6-O déméthylases est indétectable, dans laquelle la plante modifiée a une teneur en thébaïne qui est d'au moins 90 % de la teneur totale en alcaloïdes extraits de latex ou de paille séchée comparée à une plante *Paperver somniferum* de type sauvage comprenant des gènes fonctionnels codant pour des codéine 3-0-déméthylases et thébaïne 6-O-déméthylases.

2. Plante *Papaver somniferum* selon la revendication 1, dans laquelle ladite délétion génomique comprend une délétion de tout ou partie de la séquence nucléotidique présentée dans SEQ ID N° : 1, dans laquelle ladite délétion comprend tout ou partie desdits trois gènes de codéine 3-0-déméthylase.

3. Plante *Papaver somniferum* selon la revendication 2, dans laquelle ladite délétion génomique comprend une délétion d'au moins 50 % de la séquence nucléotidique présentée dans SEQ ID N° : 1, dans laquelle ladite délétion comprend tout ou partie desdits trois gènes de codéine 3-0-déméthylase.

4. Plante *Papaver somniferum* selon les revendications 1 à 3, dans laquelle ladite délétion génomique comprend une délétion d'au moins 50 % de la séquence nucléotidique présentée dans SEQ ID N° : 25, dans laquelle ladite modification est une délétion de tout ou partie desdits cinq ou plus gènes de thébaine 6-0-déméthylase.

5. Plante *Papaver somniferum* selon la revendication 4, dans laquelle ladite modification génomique comprend une délétion de la séquence nucléotidique présentée dans SEQ ID N° : 25 dans laquelle ladite plante *Papaver somniferum* subit une délétion de cinq gènes de thébaïne 6-0-déméthylase.

6. Plante *Papaver somniferum* selon la revendication 5, dans laquelle la plante *Papaver* comprend une teneur en thébaïne qui est d'au moins 91 %, 92 %, 93 %, 94 %, 95 %, 96 %, 97 %, 98 % ou 99 % de la teneur totale en alcaloïdes extraits de latex ou de paille séchée.

7. Plante *Papaver somniferum* selon l'une quelconque des revendications 1 à 6, dans laquelle la plante *Papaver* comprend une teneur en thébaïne d'au moins 1,0 % en poids de la paille séchée de ladite plante modifiée.

8. Plante *Papaver somniferum* selon la revendication 7, dans laquelle la teneur en thébaïne de ladite plante *Papaver* est entre 1,0 % en poids et 10,0 % en poids de la paille séchée.

9. Plante *Papaver somniferum* selon la revendication 8, dans laquelle la teneur en thébaïne de ladite plante *Papaver* est entre 5,0 % en poids et 6,0 % en poids de la paille séchée.

10. Plante *Papaver somniferum* selon la revendication 9, dans laquelle la teneur en thébaïne de ladite plante *Papaver* est d'environ 5,7 % en poids de la paille séchée.

11. Graine ou gousse de graine obtenue ou pouvant être obtenue à partir d'une plante *Papaver somniferum* selon l'une quelconque des revendications 1 à 10.

12. Paille séchée obtenue ou pouvant être obtenue à partir de la plante *Papaver somniferum* selon l'une quelconque des revendications 1 à 10.

13. Procédé d'extraction de thébaïne à partir d'une plante *Papaver somniferum* comprenant les étapes :
i) de récolte d'une plante ou d'une matière végétale à partir d'une plante selon l'une quelconque des revendications 1 à 10 ;
ii) de formation d'un mélange réactionnel de matière végétale particulaire ;
iii) d'extraction par solvant du mélange réactionnel pour obtenir une fraction enrichie en alcaloïdes ; et
iv) de concentration de ladite fraction enrichie en alcaloïdes pour obtenir une fraction enrichie en thébaïne.

14. Procédé selon la revendication 13, dans lequel ladite matière végétale comprend de la paille de pavot.

15. Procédé selon la revendication 13, dans lequel ladite matière végétale comprend du latex de pavot.
